# EUROPEAN PATENT APPLICATION

(11) **EP 2 011 564 A1**
(43) Date of publication of application: **07.01.2009**
(21) Application number: 07013243.6
(22) Date of filing: 06.07.2007
(51) Int. Cl.: B01J 3/04, C07C 29/151

(54) **Process and plant for synthesis of methanol with H2 recovery from purge of synthesis loop**

(71) Applicant: METHANOL CASALE S.A., 6900 Lugano-Besso (CH)
(72) Inventor: Filippi, Ermanno, 6976 Castagnola (CH)
(74) Representative: Zardi, Marco

(57) **Abstract**

Process and plant for the synthesis of methanol with hydrogen recovery from the synthesis loop (1), in which: the synthesis loop operates at the production pressure of the fresh make-up gas (6); a drawing is provided of purge gas (14) from the unreacted recycle gases, said drawing being downstream of the circulation compression section (5) of the synthesis loop (1); the recovery hydrogen (16) is obtained with a low pressure drop system (15), and is recycled directly into the synthesis loop (1) upstream of said compression section.

## Description

### Field of application

The present invention, in its broadest form, relates to the technical field of processes and plants for the synthesis of methanol. More in detail, the invention relates to a process and plant for the synthesis of methanol with hydrogen recovery from the purge of the synthesis loop.

With this term, it is intended a methanol synthesis process in a synthesis loop comprising at least the steps of: feeding a reagent gas flow to a methanol synthesis section, obtaining a flow of reaction products, feeding said reaction product flow to a condensation and separation section, obtaining a crude liquid methanol flow and a flow of unreacted recycled gases; drawing a purge gas flow from said recycle gas flow, with recovery and reinsertion, in the synthesis loop, of the hydrogen therein contained, and feeding the recycle gas flow to a circulation compression section of the synthesis loop.

### Prior Art

According to conventional technique, methanol is produced starting from a syngas containing hydrogen (H2) and carbon monoxide (CO), which is reacted at high temperature (200-300 °C) and pressure (40 - 150 bar) in the presence of an appropriate catalyst. The gaseous reaction products are then cooled, obtaining methanol in liquid state and a flow of unreacted gases; these latter are recycled to the synthesis section after a purge of methane and inerts.

A typical plant which operates according to such process essentially comprises: a synthesis section, constituted for example by a synthesis reactor or plurality of reactors in parallel; a preheating section of the reagent gas, by means of heat recovery from the reaction products; a condensation and separation section where the liquid methanol and the gases to be recycled are obtained. The synthesis section, the preheating section and the condensation-separation section substantially operate at the same pressure forming the so-called synthesis loop.

The "fresh" syngas, also called make-up gas, is fed to the synthesis loop by means of a suitable supply compressor, which raises the gas pressure from the production value up to the operating pressure of the synthesis loop.

The gaseous recycle flow, exiting the condensation and separation section, is fed to a circulation compressor and subsequently to the preheating section and synthesis section. The purge drawing point is located, according to the prior art, upstream (suction side) of said circulation compressor.

It is also known to recover the hydrogen contained in the purge gas, by means of devices such as molecular sieves or membranes, obtaining a recovery hydrogen flow which is reinserted into the synthesis loop by mixing it with the reagent gas flow. This technique is particularly advantageous in plants using reagent gases from carbon fuel gasification, said gases having a high carbon monoxide content (CO). Syngas with high CO concentration, greater than that required by the stoichiometry of the reaction, is obtained for example by the gasification of coal, coke, oil or other carbon residues.

A high CO content in the syngas leads to some technical difficulties, including increased speed of the synthesis reaction, with risk of reactor overheating, and greater risk of formation of by-products. The hydrogen recovery from the purge, and related hydrogen enrichment of the syngas fed to the reactor, allows to remedy the above difficulties.

The recovery of hydrogen by aforementioned devices, however, involves a pressure drop (tens of bars in the case of the membranes) and, with known synthesis loop configurations, provides the hydrogen at a lower pressure than that of the synthesis loop. There follows the need to recompress the hydrogen flow, and such task is currently performed by an additional compressor or through the supply compressor itself.

Adding a compressor to the plant is a disadvantage, because of cost and energy consumption of such equipment, as well as addition of a possible cause of unreliability. It is clear, in fact, that a failure of the hydrogen compressor can compromise the working of the whole plant.

To this purpose, it should be noted that in many applications the fresh syngas is available at a pressure of 50-60 bar, sufficient to carry out the synthesis reaction. In these cases, the supply compressor could in theory be eliminated, accepting to operate the synthesis loop at the relatively low syngas production pressure. By operating with CO-rich gas, one would have the further advantage of reducing the risk of overheating, since the reaction speed depends on the pressure. There is therefore a strong incentive to eliminate the supply compressor, by operating the synthesis loop at the gasification pressure itself, particularly for the production of methanol from CO-rich gas.

Nevertheless, due to the above problem of the pressure drop in the hydrogen recovery system, advantages from the possible elimination of the supply compressor, also in terms of improved reliability, would be voided by the need to provide, in any case, a compressor for the hydrogen. Hence, it is generally preferred to keep the synthesis loop at high pressure (80 - 90 bar) and to compress the hydrogen through the supply compressor. This choice is often also motivated by the use of tube bundle reactors, whose productivity would be significantly affected by pressure lowering.

Hydrogen separation systems are also known with low pressure drop (typically a few bars), including the PSA (Pressure Swing Adsorption) systems, but their use would not solve the above problem, still leaving the need to recompress the hydrogen.

In summary, with reference to methanol synthesis with hydrogen recovery from the synthesis loop purge, in particular from CO-rich gas, the prior art teaches a plant configuration wherein the synthesis loop operates at a pressure greater than the gasification pressure, a supply compressor being thus provided; the purge gas, inside the synthesis loop, is drawn upstream of the circulation compressor and sent to a membrane, molecular sieve or equivalent system to recover the hydrogen portion; the so-obtained hydrogen flow is fed to the supply compressor, to raise it to the operating pressure of the synthesis loop.

### Summary of the invention

The problem underlying the present invention is to devise and make available a process for methanol synthesis, with recovery of the hydrogen from the synthesis loop, which solves the above-listed problems. In particular, the problem underlying the invention is to find a way to operate the synthesis loop at the same pressure at which the make-up gas is available, eliminating both the supply compressor and the additional compressor for the hydrogen.

The problem is solved with a methanol synthesis process in a synthesis loop comprising the steps of: feeding a reagent gas flow to a methanol synthesis section, with obtainment of a reaction product flow; feeding said reaction product flow to a condensation and separation section, with obtainment of a crude liquid methanol flow and a flow of unreacted recycle gases; drawing a flow of purge gases from the synthesis loop, with recovery and reinsertion, in the synthesis loop, of the hydrogen therein contained, and feeding said recycle gas flow to a circulation compression section of the synthesis loop; characterised by the steps of:
- drawing said purge gas flow downstream of said compression section;
- feeding said purge gas flow to a low pressure drop separation system, with obtainment of a recovery hydrogen gas flow substantially at the same suction pressure of the compression section;
- recycling said recovery hydrogen flow in the synthesis loop, with mixing with the recycling gas flow exiting from the condensation and separation section;
- feeding a flow of fresh reagent make-up gases directly into the synthesis loop, downstream of said purge gas flow, said synthesis loop being operated substantially at the same production pressure of the fresh reagent gases.

Preferably, the low pressure drop separation system is a per se known PSA molecular sieve.

The reagent gases are preferably fed to the reaction section through a preheating section, which recovers the heat of the reaction products.

A plant for methanol synthesis adapted to operate according to the process of claim 1 is also an object of the invention, having a synthesis loop which comprises at least a methanol synthesis section, a condensation and separation section of the synthesis products, adapted to obtain a crude liquid methanol flow and a flow of unreacted recycle gases, a system for purging said recycle gas flow and recovering the hydrogen therein contained, and a circulation compressor of the synthesis loop, characterised by comprising:
- at least one drawing branch of a purge gas flow downstream of said circulation compressor;
- a low pressure drop separation system, fed by said purge gas drawing branch, adapted to obtain a recovery hydrogen flow substantially at the same suction pressure of the circulation compressor;
- an insertion branch of said recovery hydrogen flow into the synthesis loop circuit, with mixing with the recycle gas flow upstream of said circulation compressor;
- a supply line of reagent make-up gases directly into the circuit of the synthesis loop and downstream of said purge gas drawing branch, said synthesis loop operating substantially at the same production pressure of said make-up gases.

Preferably, the synthesis section comprises a pseudo-isothermal reactor equipped with an internal plate heat exchanger unit, for example as disclosed in EP-1436075 or EP-1757360. The adoption of such reactor allows to reach large production capacity even at the relatively low pressure (for example 50 - 60 bars) of the synthesis loop, which is due to operating the synthesis loop at the same gasification pressure.

The advantages of the invention are the following. The supply compressor is eliminated, thanks to the synthesis loop operating at the same production pressure of the make-up gas; the additional hydrogen compressor is also eliminated, due to the purge gas drawing downstream of the circulation compressor, in combination with the adoption of a low pressure drop system. The latter in fact makes available the hydrogen substantially at the suction pressure of the aforesaid compressor, and the hydrogen can be reinserted in the circuit of the synthesis loop without any further compression.

It follows that the plant is simpler, has lower energy consumption and is more reliable.

### Brief description of the drawings

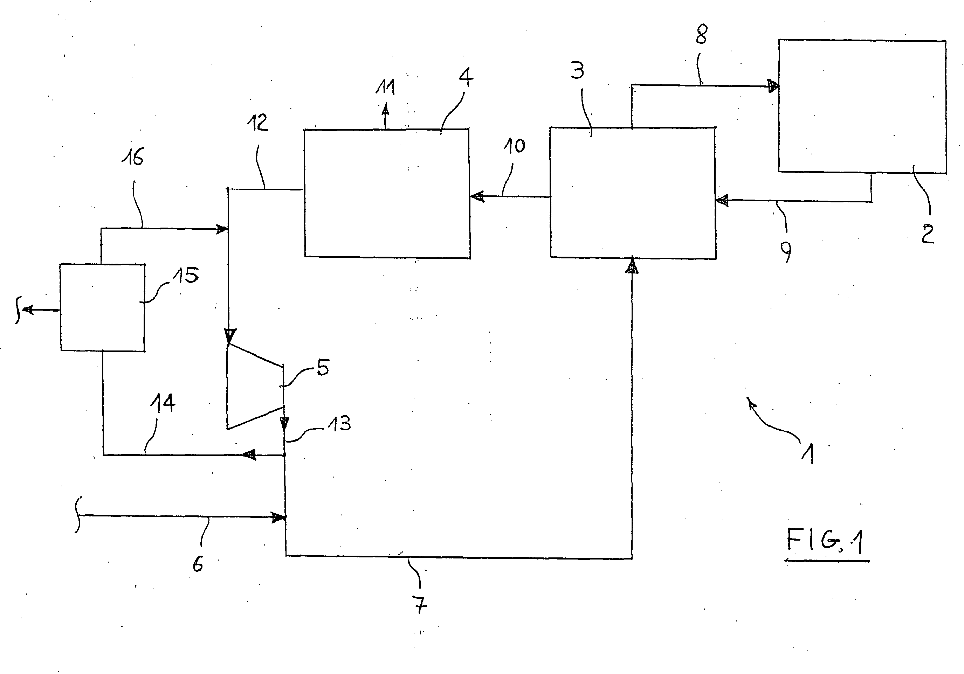

Fig. 1 shows, in a simplified manner, a diagram of the process in accordance with the invention.

### Detailed description

With reference to Fig. 1, a process diagram is shown for the production of methanol which comprises a synthesis loop 1 comprising: a synthesis section 2, a preheating section 3 of the reagent gases, a condensation-separation section 4, and a circulation compressor 5.

The synthesis loop 1 is fed for example by a gasification section of carbon fuel with oxygen (not shown), producing a flow 6 of fresh gas or make-up gas essentially composed of a mixture of CO, CO2, H2, CH4.

The make-up gas flow 6, as illustrated, is fed directly into the synthesis loop 1, that is with no compression of said flow 6 before insertion in the loop 1. In other words, the make-up gas flow 6 is fed directly at the gasification pressure, for example about 60 bars, and the synthesis loop 1 operates at this same pressure.

The synthesis section 2 is fed by a reagent gas flow 7, by means of the preheating section 3 providing a preheated reagent gas flow 8, recovering heat from reaction products (flow line 9) exiting the same synthesis section 2.

The cooled reaction products, indicated by the flow line 10, are fed to the condensation-separation section 4, obtaining a flow 11 of crude liquid methanol, for subsequent purification and distillation, and a flow 12 of recycle gas substantially containing CO, CO2, H2, CH4 and possible incondensable gases.

Said flow 12 of recycle gas is fed to the circulation compressor 5. The gaseous delivery flow of said compressor 5, indicated by the flow line 13, is mixed with the make-up gas flow 6, producing the reagent flow 7.

A gas purge is provided downstream of the compressor 5, that is from flow 13 exiting the compressor itself, thus obtaining a flow 14 of purge gas. Said purge gas flow 14 is fed to a separation device 15 of molecular sieve type, for example PSA (Pressure Swing Adsorption), which is per se known, capable of separating the hydrogen (H2) from other gases present in the purge flow 14.

Said device 15 obtains a flow 16 essentially composed of recovered hydrogen at a pressure slightly less than that of the flow 13 exiting the compressor 5, and substantially equal to the pressure of the flow 12 of recycle gases obtained from the section 4. Said recovery hydrogen flow 13 is reinserted into the synthesis loop 1, upstream the compressor 5, by mixing it with the flow 12 exiting the section 4 and fed to the same compressor 5.

It must be furthermore noted that the purge flow 14 is drawn upstream the insertion of make-up gas flow 6, i.e. the make-up gas is added downstream of the purge. In this manner, purging of the fresh gas flow 6, as well as diluting the purge gas flow 14 with fresh gas, which would lower the hydrogen recovery efficiency of the device 15, are avoided.

Preferably, the synthesis section 2 comprises a plate synthesis reactor, which permits high production rate even at the relatively low pressure of the synthesis loop set by the gasification section, i.e. by the fact that no compressor of the make-up flow 6 is provided. With the term plate reactor, more precisely, it is intended a reactor equipped with internal plate heat exchanger (cooling) unit, immersed in a catalytic mass, to keep the reaction temperature substantially constant.

The preheating section 3 can be constituted of an appropriate heat exchanger, and the section 4 can be constituted by a condenser-separator.

A plant adapted to achieve the above-described process, therefore, comprises at least one synthesis loop 1 as described above, and appropriate connection means between the components corresponding to the flow lines of Fig. 1. In particular, the plant comprises a separation device 15 of molecular sieve type, for example PSA, which is arranged in parallel to the circulation compressor 5, with a drawing branch of the purge gas corresponding to the flow line 14, the drawing point being downstream (pressure side) of said compressor 5, and a reinsertion branch of recovered hydrogen into the synthesis loop 1 corresponding to flow line 16, the reinsertion point being upstream (suction side) the same compressor 5.

The pressure drop through device 15, on the order of a few bars, substantially corresponds to the pressure difference between suction and pressure side of the compressor 5; therefore it is possible to operate the purge drawing and hydrogen recovery as described, without additional compressor units.

The invention thus attains the above objects, allowing the operation of the synthesis loop at the same production pressure of the make-up gas (gasification), and recovering the hydrogen contained in the purge gas without the need for compression.

## Claims

1. Methanol synthesis process in a synthesis loop (1) comprising the steps of: feeding a reagent gas flow (8) to a methanol synthesis section (2), obtaining a reaction product flow (9); feeding said reaction product flow to a condensation and separation section (4), with obtainment of a crude liquid methanol flow (11) and a flow (12) of unreacted recycle gases; drawing a purge gas flow (14) from the synthesis loop (1), with recovery and reinsertion, in the synthesis loop (1), of the hydrogen therein contained, and feeding said recycle gas flow (12) to a circulation compression section (5) of the synthesis loop; **characterised by** the steps of:
- drawing said purge gas flow (14) downstream of said compression section (5);
- feeding said purge gas flow (14) to a low pressure drop separation system (15), obtaining a gaseous flow (16) of recovered hydrogen substantially at the same suction pressure of the compression section (5);
- recycling said recovered hydrogen flow (16) in the synthesis loop (1), by mixing with the recycle gas flow (12) exiting the condensation and separation section (4), upstream of said compression section (5);
- feeding a flow of fresh reagent make-up gases (6) directly into the synthesis loop (1), downstream of the drawing of said purge gas flow (14), said synthesis loop (1) operating substantially at the same production pressure of the fresh reagent gases.

2. Process according to claim 1, **characterised in that** said low pressure drop separation system (15) comprises a molecular sieve.

3. Process according to claim 1 or 2, **characterised in that** the synthesis loop (1) operates at a pressure of about 60 bars.

4. Plant for the synthesis of methanol adapted to operate according to the process of claim 1, having a synthesis loop (1) which comprises at least one methanol synthesis section (2), a condensation and separation section (4) of the synthesis products, adapted to obtain a crude liquid methanol flow (11) and a flow (12) of unreacted recycle gases, a system (14, 15, 16) for purging said recycle gas flow (12) and recovering the hydrogen therein contained, and a circulation compressor (5) of the synthesis loop, **characterised in that** it comprises:
- at least one drawing branch (14) of a purge gas flow placed downstream of said circulation compressor;
- a low pressure drop separation system (15), fed by said purge gas drawing branch, adapted to obtain a recovery hydrogen flow substantially at the same suction pressure of the circulation compressor;
- an insertion branch (16) of said recovery hydrogen flow into the circuit of the synthesis loop, with mixing with the recycle gas flow, upstream of said circulation compressor;
- a supply line (6) of reagent make-up gases, directly into the circuit of the synthesis loop (1) and downstream of said purge gas drawing branch (14), said synthesis loop (1) operating substantially at the same production pressure of said make-up gases.

5. Plant according to claim 4, **characterised in that** the synthesis section (2) comprises at least one reactor equipped with an internal plate heat exchanger unit.
